(19) Europäisches Patentamt
European Patent Office
Office européen des brevets

(11) Veröffentlichungsnummer: **0 008 334**
**B1**

(12) # EUROPÄISCHE PATENTSCHRIFT

(45) Veröffentlichungstag der Patentschrift: **14.10.81**

(51) Int. Cl.³: **C 07 C 69/743**, A 01 N 53/00, C 07 C 119/00

(21) Anmeldenummer: **79102088.6**

(22) Anmeldetag: **25.06.79**

(54) **Alpha-phenyl-alpha-cyclopropylessigsäureester, ihre Herstellung und Verwendung als Schädlingsbekämpfungsmittel.**

(30) Priorität: **26.06.78 CH 6937/78**
**19.12.78 CH 12885/78**
**12.04.79 CH 3532/79**

(43) Veröffentlichungstag der Anmeldung: **05.03.80 Patentblatt 80/5**

(45) Bekanntmachung des Hinweises auf die Patenterteilung: **14.10.81 Patentblatt 81/41**

(84) Benannte Vertragsstaaten: **AT BE CH DE FR GB IT NL**

(56) Entgegenhaltungen: **DE-A-2 717 414**

(73) Patentinhaber: **CIBA-GEIGY AG, Patentabteilung Postfach, CH-4002 Basel (CH)**

(72) Erfinder: **Drabek, Jozef, Dr., Benkenstrasse 12, CH-4104 Oberwil (CH)**
Erfinder: **Ackermann, Peter, Dr., Reichensteinerstrasse 12, CH-4153 Reinach (CH)**
Erfinder: **Farooq, Saleem, Dr., Im Schalengarten 2, CH-4107 Ettingen (CH)**
Erfinder: **Gsell, Laurenz, Dr., Maiengasse 56, CH-4056 Basel (CH)**
Erfinder: **Kristiansen, Odd, Dr., Delligrabenstrasse 7, CH-4313 Möhlin (CH)**
Erfinder: **Wehrli, Rudolf, Dr., Dianastrasse 2, CH-4310 Rheinfelden (CH)**

(74) Vertreter: **Zumstein sen., Fritz, Dr. et al, Bräuhausstrasse 4, D-8000 München 2 (DE)**

EP 0 008 334 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Alpha-phenyl-alpha-cyclopropylessigsäureester,
ihre Herstellung und Verwendung als Schädlingsbekämpfungsmittel

Die vorliegende Erfindung betrifft α-Phenyl-α-cyclopropyl-acetate, Verfahren zu ihrer Herstellung und ihre Verwendung in der Schädlingsbekämpfung. In der deutschen Offenlegungsschrift 2 717 414 werden analoge Verbindungen und Verfahren zu ihrer Herstellung beschrieben, die aber keine akarizide Wirkung besitzen.

Die erfindungsgemäßen α-Phenyl-α-cyclopropyl-acetate sind demgegenüber neu und haben die Formel

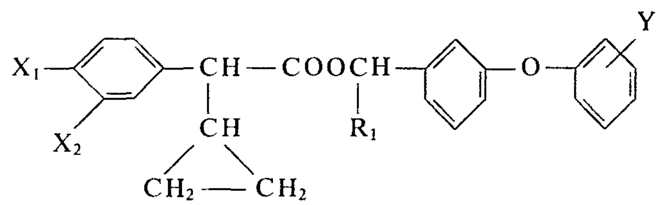

(I)

worin $X_1$ Wasserstoff, Halogen oder $C_1-C_4$-Alkyl, $X_2$ Wasserstoff, Methyl oder Halogen, $R_1$ Wasserstoff, Cyano, Vinyl oder Äthinyl und Y Fluor oder Brom bedeuten.

Unter Halogen ist dabei Fluor, Chlor, Brom oder Jod, insbesondere aber Chlor, zu verstehen.

Bei $X_1$ kommen beispielsweise als Alkylgruppen in Frage: Methyl, Äthyl, Propyl, Isopropyl, n-, i-, sek.-, tert.-Butyl. Wegen ihrer Wirkung bevorzugt sind Verbindungen der Formel I, worin $X_1$ Wasserstoff, Chlor oder Methyl, $X_2$ Wasserstoff oder Chlor, $R_1$ Wasserstoff, Cyano, Vinyl oder Äthinyl und Y Fluor oder Brom bedeuten.

Insbesondere bevorzugt sind aber Verbindungen der Formel I, worin $X_1$ Chlor oder Methyl, $X_2$ Wasserstoff, $R_1$ Cyano oder Äthinyl und Y o-Fluor, m-Fluor oder p-Fluor bedeuten.

Die Verbindungen der Formel I können nach an sich bekannten Methoden, z. B. wie folgt hergestellt werden.

1) 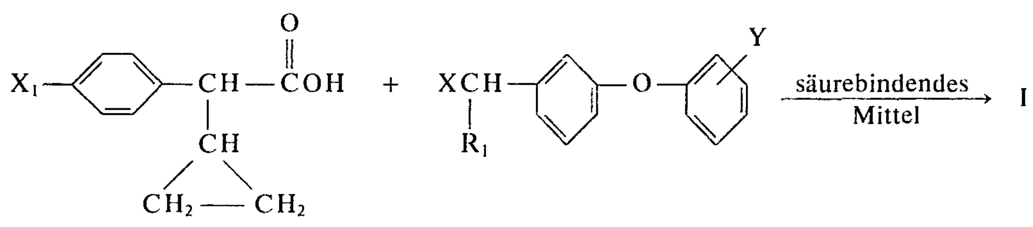


(II) (III)

2) 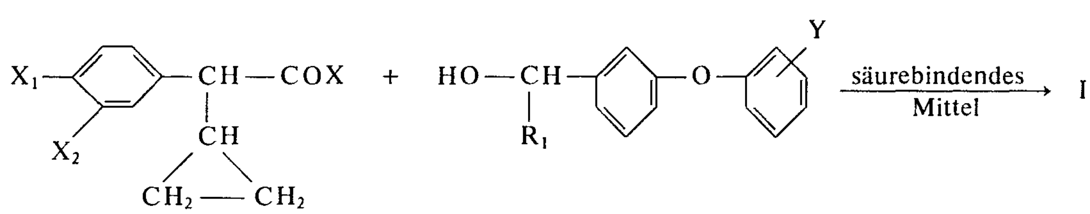


(IV) (V)

3) 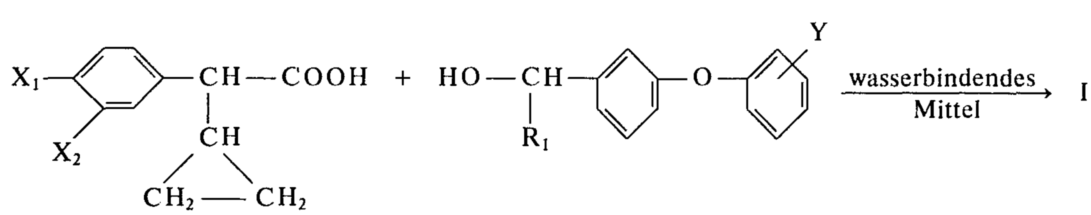


(II) (V)

4) 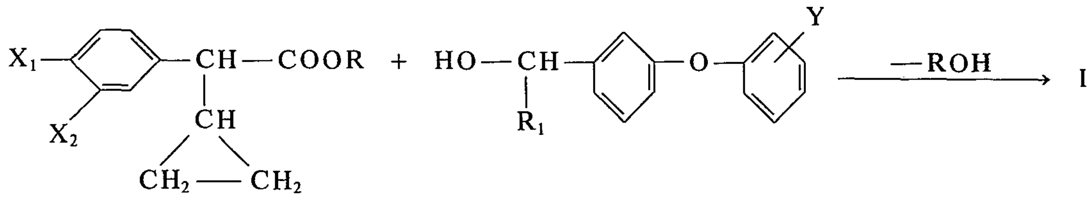

(VI) (V)

In den Formeln II bis VI haben $X_1$, $X_2$, Y und $R_1$ die für die Formel I angegebene Bedeutung.

In den Formeln III und IV steht X für ein Halogenatom, insbesondere Chlor oder Brom, und in der Formel VI steht R für $C_1-C_4$-Alkyl, insbesondere für Methyl oder Äthyl. Als säurebindendes Mittel für die Verfahren 1 und 2 kommen insbesondere tertiäre Amine, wie Trialkylamin und Pyridin, ferner Hydroxide, Oxide, Carbonate und Bicarbonate von Alkali- und Erdalkalimetallen sowie Alkalimetallalkoholate wie z. B. Kalium-t.-butylat und Natriummethylat in Betracht. Als wasserbindendes Mittel für das Verfahren 3 kann z. B. Dicyclohexylcarbodiimid verwendet werden. Die Verfahren 1 bis 4 werden bei einer Reaktionstemperatur zwischen −10 und 120°C, meist zwischen 20 und 80°C bei normalem oder erhöhtem Druck und vorzugsweise in einem inerten Lösungs- oder Verdünnungsmittel durchgeführt. Als Lösungs- oder Verdünnungsmittel eignen sich z. B. Äther und ätherartige Verbindungen wie Diäthyläther, Dipropyläther, Dioxan, Dimethoxyäthan und Tetrahydrofuran; Amide wie N,N-dialkylierte Carbonsäureamide; aliphatische, aromatische sowie halogenierte Kohlenwasserstoffe, insbesondere Benzol, Toluol, Xylole, Chloroform und Chlorbenzol; Nitrile wie Acetonitril; Dimethylsulfoxid und Ketone wie Aceton und Methyläthylketon.

Die Ausgangsstoffe der Formeln II bis VI sind bekannt oder können analog bekannten Methoden hergestellt werden.

Die Verbindungen der Formel I liegen als Gemisch von verschiedenen optisch aktiven Isomeren vor, wenn bei der Herstellung nicht einheitlich optisch aktive Ausgangsmaterialien verwendet wurden. Die verschiedenen Isomerengemische können nach bekannten Methoden in die einzelnen Isomeren aufgetrennt werden. Unter der Verbindung der Formel I versteht man sowohl die einzelnen Isomeren, als auch deren Gemische.

Die Verbindungen der Formel I eignen sich zur Bekämpfung von verschiedenartigen tierischen und pflanzlichen Schädlingen.

Insbesondere eignen sich die Verbindungen der Formel I zur Bekämpfung von Insekten, phytopathogenen Milben und von Zecken z. B. der Ordnungen Lepidoptera, Coleoptera, Homoptera, Heteroptera, Diptera, Acarina, Thysanoptera, Orthoptera, Anoplura, Siphonaptera, Mallophaga, Thysanura, Isoptera, Psocoptera und Hymenoptera.

Vor allem eignen sich Verbindungen der Formel I zur Bekämpfung von pflanzenschädigenden Insekten, insbesondere pflanzenschädigenden Fraßinsekten, in Zier- und Nutzpflanzen, insbesondere in Baumwollkulturen (z. B. gegen Spodoptera littoralis und Heliothis virescens) und Gemüsekulturen (z. B. gegen Leptinotarsa decemlineata und Myzus persicae).

Wirkstoffe der Formel I zeigen auch eine sehr günstige Wirkung gegen Fliegen wie z. B. Musca domestica und Mückenlarven.

Überraschenderweise haben Verbindungen der Formel I ein breiteres, insektizides Wirkungsspektrum als chemisch analoge, aus der DOS Nr. 2 717 414 bekannte Verbindungen.

Die akarizide bzw. insektizide Wirkung läßt sich durch Zusatz von anderen Insektiziden und/oder Akariziden wesentlich verbreitern und an gegebene Umstände anpassen. Als Zusätze eignen sich z. B. org. Phosphorverbindungen; Nitrophenole und deren Derivate; Formamidine; Harnstoffe; andere pyrethrinartige Verbindungen sowie Karbamate und chlorierte Kohlenwasserstoffe.

Mit besonderem Vorteil werden Verbindungen der Formel I auch mit Substanzen kombiniert, welche einen synergistischen oder verstärkenden Effekt auf Pyrethroide ausüben. Beispiele solcher Verbindungen sind u. a. Piperonylbutoxid, Propinyläther, Propinyloxime, Propinylcarbamate und Propinylphosphate, 2-(3,4-Methylendioxyphenoxy)-3,6,9-trioxaundecan (Sesamex resp. Sesoxane), S,S,S-Tributylphosphorotrithioate, 1,2-Methylendioxy-4-(2-(oxtylsulfinyl)-propyl)-benzol.

Verbindungen der Formel I können für sich allein oder zusammen mit geeigneten Trägern und/oder Zuschlagstoffen eingesetzt werden. Geeignete Zuschlagstoffe können fest oder flüssig sein und entsprechen den in der Formulierungstechnik üblichen Stoffen wie z. B. natürlichen oder regenerierten Stoffen, Lösungs-, Dispergier-, Netz-, Haft-, Verdickungs-, Binde- und/oder Düngemitteln.

Die Herstellung erfindungsgemäßer Mittel erfolgt in an sich bekannter Weise durch inniges Vermischen und/oder Vermahlen der Wirkstoffe der Formel I mit den geeigneten Trägerstoffen, gegebenenfalls unter Zusatz von gegenüber den Wirkstoffen inerten Dispergier- oder Lösungsmitteln. Die Wirkstoffe können in den folgenden Aufarbeitungsformen vorliegen und angewendet werden:

Feste Aufarbeitungsformen:

Stäubemittel, Streumittel, Granulate (Umhüllungsgranulate, Imprägnierungsgranulate und Homogengranulate);

Flüssige Aufarbeitungsformen:

a) in Wasser dispergierbare Wirkstoffkonzentrate: Spritzpulver (wettable powders), Pasten, Emulsionen;

b) Lösungen

Der Gehalt an Wirkstoff in den oben beschriebenen Mitteln liegt zwischen 0,1 bis 95%, dabei ist zu erwähnen, daß bei der Applikation aus dem Flugzeug oder mittels anderer geeigneter Applikationsgeräte Konzentrationen bis zu 99,5% oder sogar reiner Wirkstoff eingesetzt werden können. Die Wirkstoffe der Formel I können beispielsweise wie folgt formuliert werden (Teile bedeuten Gewichtsteile):

## Stäubemittel

Zur Herstellung eines a) 5%igen und b) 2%igen Stäubemittels werden die folgenden Stoffe verwendet:

| | | |
|---|---|---|
| a) | 5 Teile | Wirkstoff |
| | 95 Teile | Talkum |
| b) | 2 Teile | Wirkstoff |
| | 1 Teil | hochdisperse Kieselsäure |
| | 97 Teile | Talkum |

Der Wirkstoff wird mit den Trägerstoffen vermischt und vermahlen.

## Granulat

Zur Herstellung eines 5%igen Granulates werden die folgenden Stoffe verwendet:

| | |
|---|---|
| 5 Teile | Wirkstoff |
| 0,25 Teile | Epichlorhydrin |
| 0,25 Teile | Cetylpolyglykoläther |
| 3,50 Teile | Polyäthylenglykol |
| 91 Teile | Kaolin (Korngröße 0,3 – 0,8 mm) |

Die Aktivsubstanz wird mit Epichlorhydrin vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschließend das Aceton im Vakuum verdampft.

## Spritzpulver

Zur Herstellung eines a) 40%igen, b) und c) 25%igen, d) 10%igen Spritzpulvers werden folgende Bestandteile verwendet:

| | | |
|---|---|---|
| a) | 40 Teile | Wirkstoff |
| | 5 Teile | Ligninsulfonsäure-Natriumsalz |
| | 1 Teil | Dibutylnaphthalinsulfonsäure-Natriumsalz |
| | 54 Teile | Kieselsäure |
| b) | 25 Teile | Wirkstoff |
| | 4,5 Teile | Calcium-Ligninsulfonat |
| | 1,9 Teile | Champagne-Kreide/Hydroxyäthylcellulose-Gemisch (1 : 1) |
| | 1,5 Teile | Natrium-dibutyl-naphthalinsulfonat |
| | 19,5 Teile | Kieselsäure |
| | 19,5 Teile | Champagne-Kreide |
| | 28,1 Teile | Kaolin |

| | | |
|---|---|---|
| c) | 25 Teile | Wirkstoff |
| | 2,5 Teile | Isooctylphenoxy-polyäthylen-äthanol |
| | 1,7 Teile | Champagne-Kreide/Hydroxyäthylcellulose-Gemisch (1 : 1) |
| | 8,3 Teile | Natriumaluminiumsilikat |
| | 16,5 Teile | Kieselgur |
| | 46 Teile | Kaolin |

| | | |
|---|---|---|
| d) | 10 Teile | Wirkstoff |
| | 3 Teile | Gemisch der Natriumsalze von gesättigten Fettalkoholsulfaten |
| | 5 Teile | Naphthalinsulfonsäure/Formaldehyd-Kondensat |
| | 82 Teile | Kaolin |

Der Wirkstoff wird in geeigneten Mischern mit dem Zuschlagstoff innig vermischt und auf entsprechenden Mühlen und Walzen vermahlen. Man erhält Spritzpulver, die sich mit Wasser zu Suspensionen jeder gewünschten Konzentration verdünnen lassen.

## Emulgierbare Konzentrate

Zur Herstellung eines a) 10%igen, b) 25%igen und c) 50%igen emulgierbaren Konzentrates werden folgende Stoffe verwendet:

| | | |
|---|---|---|
| a) | 10 Teile | Wirkstoff |
| | 3,4 Teile | epoxydiertes Pflanzenöl |
| | 3,4 Teile | eines Kombinationsemulgators, bestehend aus Fettalkoholpolyglykoläther und Alkylarylsulfonat-Calcium-Salz |
| | 40 Teile | Dimethylformamid |
| | 43,2 Teile | Xylol |

| | | |
|---|---|---|
| b) | 25 Teile | Wirkstoff |
| | 2,5 Teile | epoxydiertes Pflanzenöl |
| | 10 Teile | eines Alkylarylsulfonat/Fettalkoholpolyglykoläther-Gemisches |
| | 5 Teile | Dimethylformamid |
| | 57,5 Teile | Xylol |

| | | |
|---|---|---|
| c) | 50 Teile | Wirkstoff |
| | 4,2 Teile | Tributylphenol-Polyglykoläther |
| | 5,8 Teile | Calcium-Dodecylbenzolsulfonat |
| | 20 Teile | Cyclohexanon |
| | 20 Teile | Xylol |

Aus solchen Konzentrationen können durch Verdünnen mit Wasser Emulsionen jeder gewünschten Konzentration hergestellt werden.

## Sprühmittel

Zur Herstellung eines a) 5%igen und b) 95%igen Sprühmittels werden die folgenden Bestandteile verwendet:

| | | |
|---|---|---|
| a) | 5 Teile | Wirkstoff |
| | 1 Teil | Epichlorhydrin |
| | 94 Teile | Benzin (Siedegrenzen 160 – 190° C) |
| b) | 95 Teile | Wirkstoff |
| | 5 Teile | Epichlorhydrin |

## Beispiel 1

Herstellung von α-Cyclopropyl-4-chlorphenylessigsäure-(3-(4-fluorphenoxy)-α-cyano-benzyl)-ester

Zur einer Lösung von 2,43 g 3-(4-fluorphenoxy)-α-cyanobenzylalkohol in 20 ml Toluol werden unter Rühren bei 0 – 10° C nacheinander 1 g Pyridin in 3 ml Toluol und 2,3 g α-Cyclopropyl-4-chlorphenylessigsäurechlorid in 5 ml Toluol zugetropft. Nach 10stündigem Rühren bei Raumtemperatur wäscht man

das Reaktionsgemisch mit Wasser, 2 n-Salzsäure und 3%iger Natriumbikarbonatlösung, trocknet die organische Schicht über Natriumsulfat und destilliert das Toluol ab. Man erhält die Verbindung der Formel

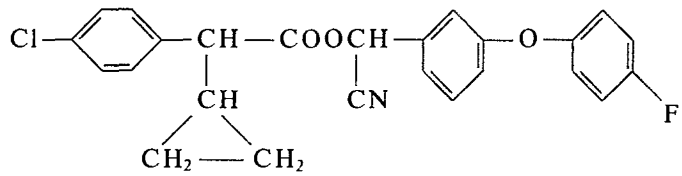

mit einem Brechungsindex von $n_D^{20} = 1,5880$

Auf analoge Weise werden auch folgende Verbindungen hergestellt:

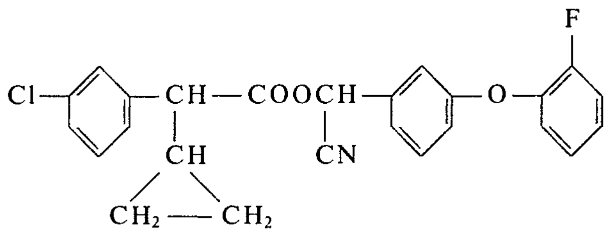

$n_D^{20} = 1,5851$

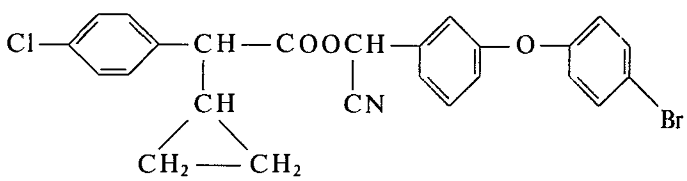

$n_D^{20} = 1,5599$

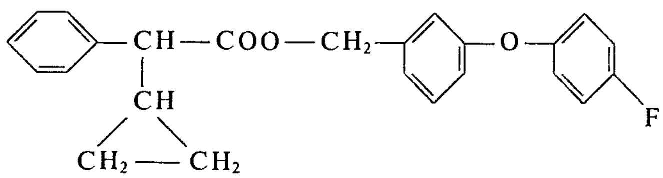

$n_D^{20} = 1,5682$

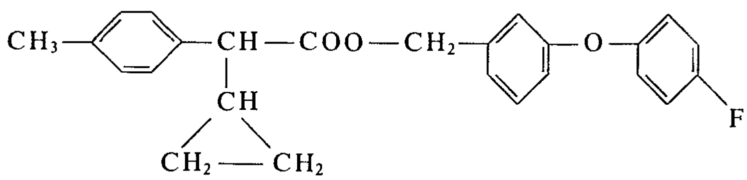

$n_D^{20} = 1,5650$

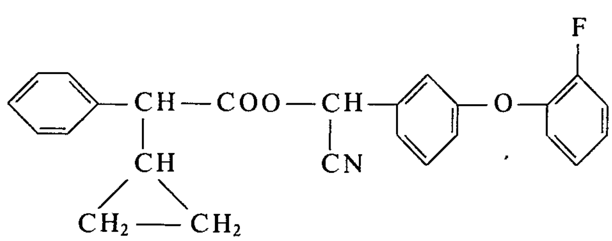

$n_D^{20} = 1,5639$

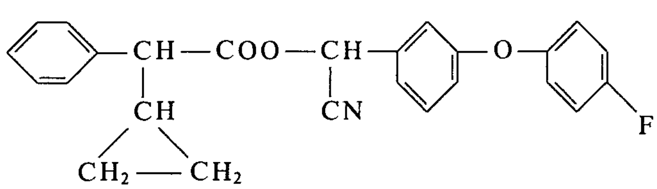

$n_D^{20} = 1,5658$

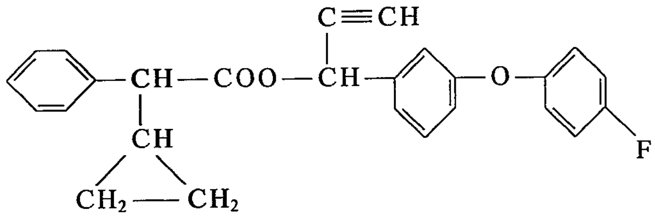

$n_D^{20^\circ} = 1{,}5678$

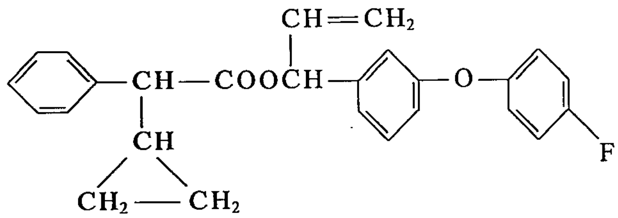

$n_D^{20^\circ} = 1{,}5638$

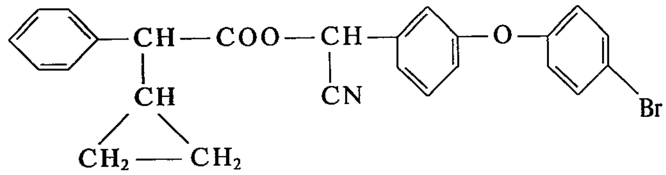

$n_D^{20^\circ} = 1{,}5913$

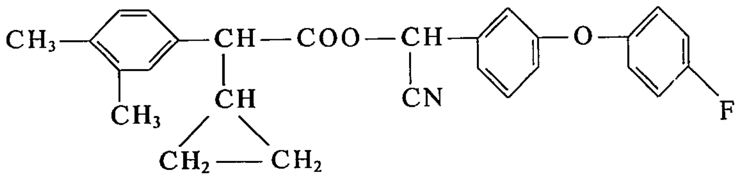

$n_D^{40^\circ} = 1{,}5571$

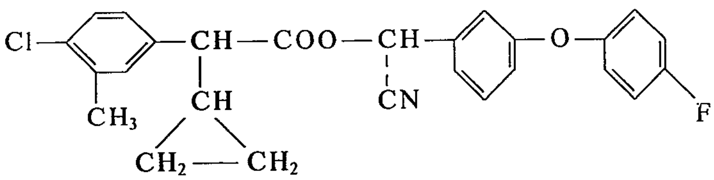

$n_D^{40^\circ} = 1{,}5646$

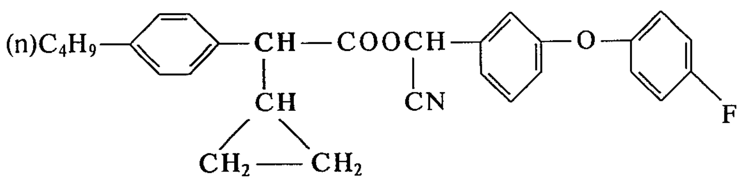

$n_D^{40^\circ} = 1{,}5460$

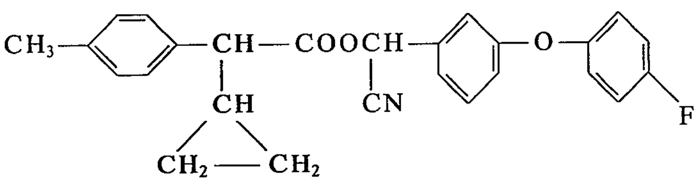

$n_D^{20^\circ} = 1{,}5627$

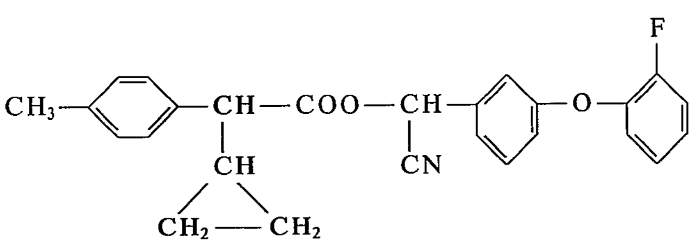

$n_D^{20^\circ} = 1{,}5625$

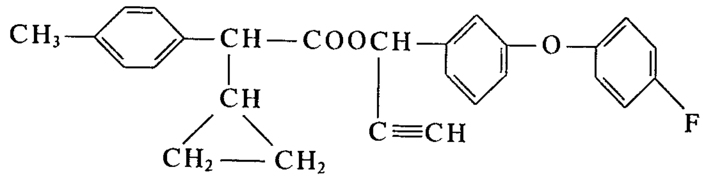

$n_D^{20°} = 1,5652$

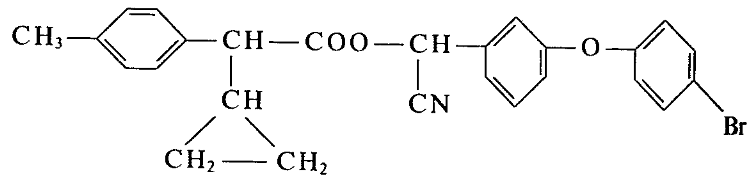

$n_D^{20°} = 1,5883$

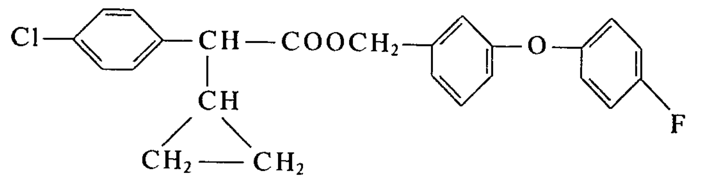

$n_D^{20} = 1,5748$

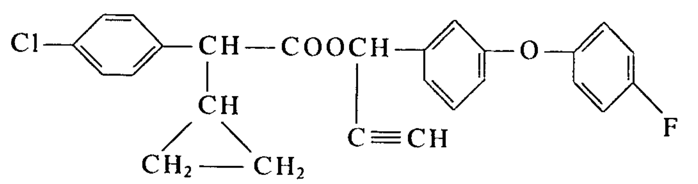

$n_D^{20} = 1,5737$

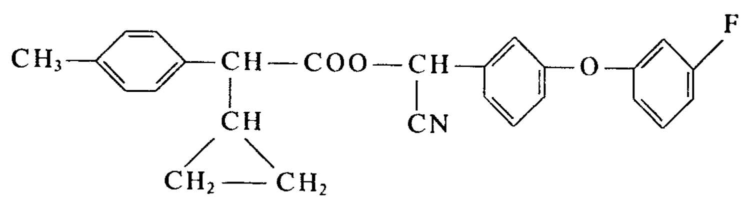

$n_D = 1,5573$

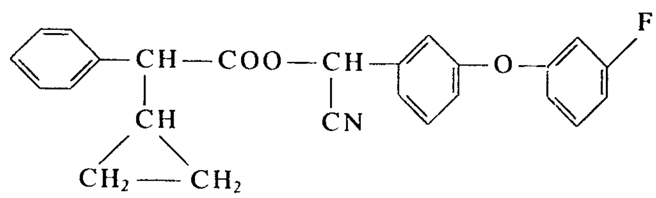

$n_D = 1,5675$

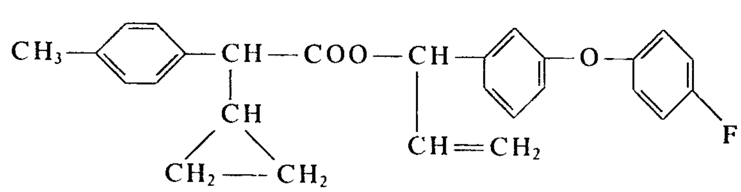

$n_D = 1,5568$

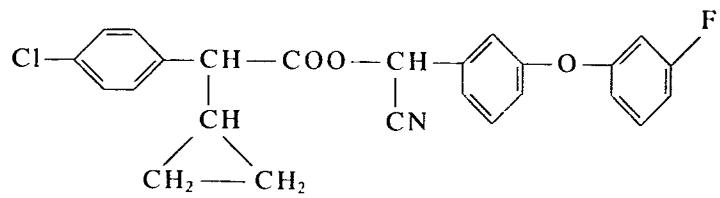

$n_D = 1,5663$

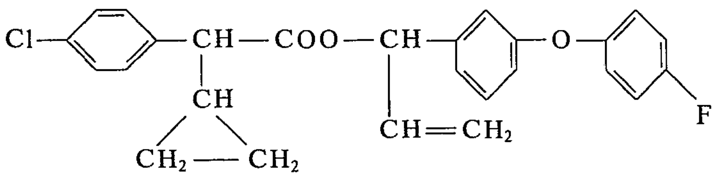

$n_D^{33°} = 1{,}5646$

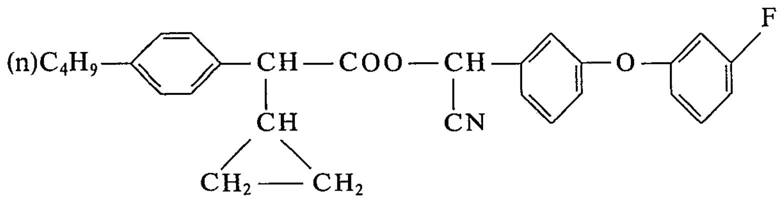

$n_D^{40°} = 1{,}5461$

Beispiel 2

A) Insektizide Fraßgift-Wirkung

Baumwollpflanzen wurden mit einer 0,05%igen wäßrigen Wirkstoffemulsion (erhalten aus einem 10%igen emulgierbaren Konzentrat) besprüht.

Nach dem Abtrocknen des Belages wurden die Baumwollpflanzen je mit Spodoptera littoralis- und Heliothis virescens-Larven $L_3$ besetzt. Der Versuch wurde bei 24° C und 60% relativer Luftfeuchtigkeit durchgeführt.

Verbindungen gemäß Beispiel 1 zeigten im obigen Test eine gute insektizide Fraßgift-Wirkung gegen Spodoptera- und Heliothis-Larven.

Beispiel 3

Akarizide Wirkung

Phaseolus vulgaris Pflanzen wurden 12 Stunden vor dem Test auf akarizide Wirkung mit einem infestierten Blattstück aus einer Massenzucht von Tetranychus urticae belegt. Die übergelaufenen beweglichen Stadien wurden aus einem Chromatographiezerstäuber mit den emulgierten Testpräparaten derart besprüht, daß kein Ablaufen der Spritzbrühe eintrat. Nach zwei und 7 Tagen wurden Larven, Adulte und Eier unter dem Binokular auf lebende und tote Individuen ausgewertet und das Ergebnis in Prozenten ausgedrückt. Während der »Haltezeit« standen die behandelten Pflanzen in Gewächshauskabinen bei 25° C.

Verbindungen gemäß Beispiel 1 wirkten im obigen Test gegen Adulte, Larven und Eier von Tetranychus urticae.

Beispiel 4

Wirkung gegen Zecken

A) Rhipicephalus bursa

Je 5 adulte Zecken bzw. 50 Zeckenlarven wurden in ein Glasröhrchen gezählt und für 1 bis 2 Minuten in 2 ml einer wäßrigen Emulsion aus einer Verdünnungsreihe mit je 100, 10, 1 oder 0,1 ppm Testsubstanz getaucht. Das Röhrchen wurde dann mit einem genormten Wattebausch verschlossen und auf den Kopf gestellt, damit die Wirkstoffemulsion von der Watte aufgenommen werden konnte.

Die Auswertung erfolgte bei den Adulten nach 2 Wochen und bei den Larven nach 2 Tagen. Für jeden Versuch liefen 2 Wiederholungen.

B) Boophilus microplus (Larven)

Mit einer analogen Verdünnungsreihe wie beim Test A) wurden mit je 20 sensiblen resp. OP-resistenten Larven Versuche durchgeführt. (Die Resistenz bezieht sich auf die Verträglichkeit von Diazinon.) Verbindungen gemäß Beispiel 1 wirken in diesen Tests gegen Adulte und Larven von Rhipicephalus bursa und sensible resp. OP-resistente Larven von Boophilus microplus.

**Patentansprüche für die Vertragsstaaten: BE, CH, DE, FR, GB, IT und NL**

1. Ein α-Phenyl-α-cyclopropyl-acetat der Formel

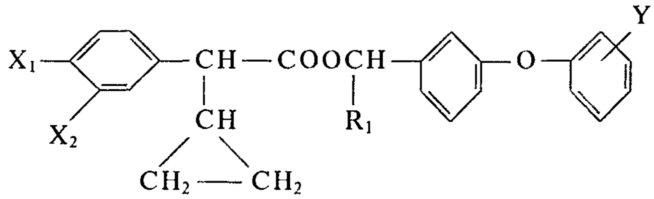

(I)

worin $X_1$ Wasserstoff, Halogen oder $C_1-C_4$-Alkyl, $X_2$ Wasserstoff, Methyl oder Halogen, $R_1$ Wasserstoff, Cyano, Vinyl oder Äthinyl und Y Fluor oder Brom bedeuten.

2. Eine Verbindung gemäß Anspruch 1, worin $X_1$ Wasserstoff, Chlor oder Methyl, $X_2$ Wasserstoff oder Chlor, $R_1$ Wasserstoff, Cyano, Vinyl oder Äthinyl und Y Fluor oder Brom bedeuten.

3. Eine Verbindung gemäß Anspruch 2, worin $X_1$ Chlor oder Methyl, $X_2$ Wasserstoff, $R_1$ Cyano oder Äthinyl und Y o-Fluor oder m-Fluor oder p-Fluor bedeuten.

4. Die Verbindung gemäß Anspruch 3 der Formel

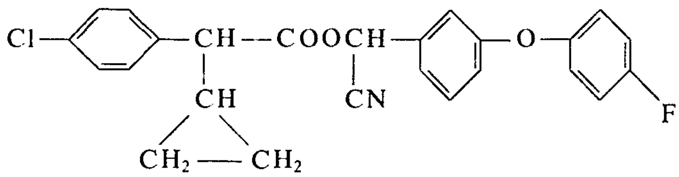

5. Die Verbindung gemäß Anspruch 3 der Formel

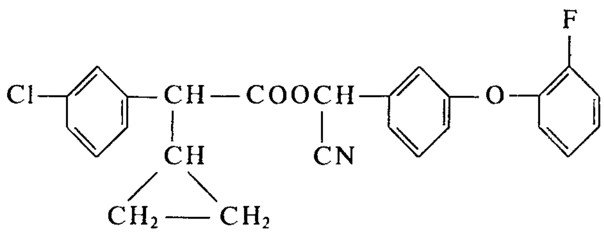

6. Verfahren zur Herstellung von Verbindungen gemäß Anspruch 1, dadurch gekennzeichnet, daß man eine Verbindung der Formel

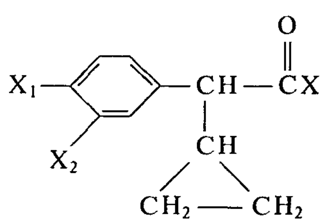

in Gegenwart eines säurebindenden Mittels mit einer Verbindung der Formel

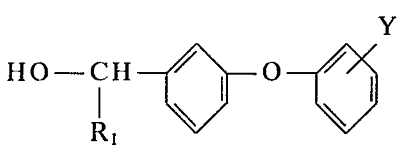

umsetzt, worin $X_1$, $X_2$, Y und $R_1$ die im Anspruch 1 angegebene Bedeutung haben und X für ein Halogenatom steht.

7. Ein Schädlingsbekämpfungsmittel, welches als aktive Komponente eine Verbindung gemäß Anspruch 1 und geeignete Träger- und/oder andere Zuschlagstoffe enthält.

8. Verwendung einer Verbindung gemäß Anspruch 1 zur Bekämpfung von verschiedenartigen tierischen und pflanzlichen Schädlingen.

9. Verwendung gemäß Anspruch 8 zur Bekämpfung von Insekten und Vertretern der Ordnung Akarina.

**Ansprüche für den Vertragsstaat: AT**

1. Ein Schädlingsbekämpfungsmittel, dadurch gekennzeichnet, daß es als aktive Komponenten ein α-Phenyl-α-cyclopropyl-acetat der allgemeinen Formel

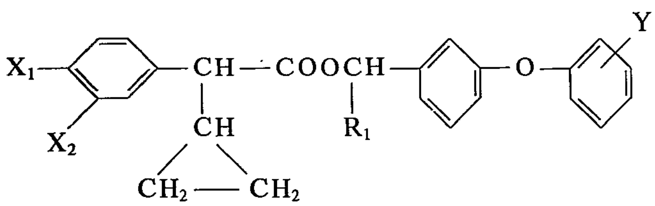

enthält, worin $X_1$ Wasserstoff, Halogen oder $C_1-C_4$-Alkyl, $X_2$ Wasserstoff, Methyl oder Halogen, $R_1$ Wasserstoff, Cyano, Vinyl oder Äthinyl und Y Fluor oder Brom bedeuten.

2. Ein Mittel gemäß Anspruch 1, worin $X_1$ Wasserstoff, Chlor oder Methyl, $X_2$ Wasserstoff oder Chlor, $R_1$ Wasserstoff, Cyano, Vinyl oder Äthinyl und Y Fluor oder Brom bedeuten.

3. Ein Mittel gemäß Anspruch 1, worin $X_1$ Chlor oder Methyl, $X_2$ Wasserstoff, $R_1$ Cyano oder Äthinyl und Y o-Fluor, m-Fluor oder p-Fluor bedeuten.

4. Ein Mittel gemäß Anspruch 1, welches als aktive Komponente die Verbindung der Formel

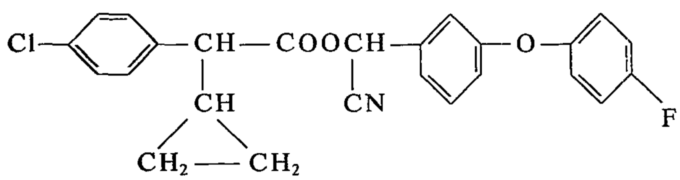

enthält.

5. Ein Mittel gemäß Anspruch 1, welches als aktive Komponente die Verbindung der Formel

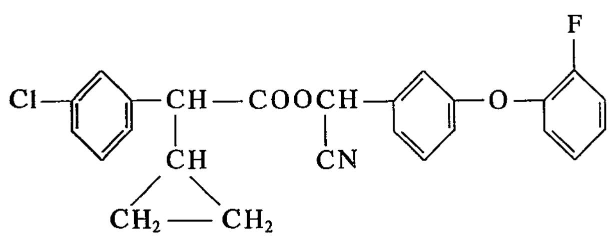

enthält.

**Claims for the contracting states: BE, CH, DE, FR, GB, IT, NL**

1. An α-phenyl-α-cyclopropylacetate of the formula

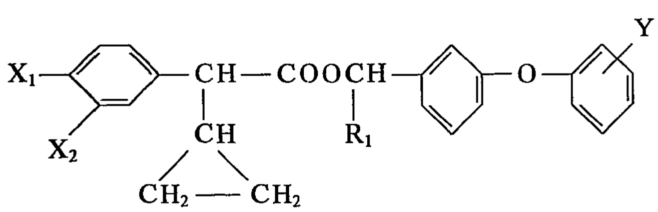

(I)

wherein $X_1$ is hydrogen, halogen or $C_1-C_4$-alkyl, $X_2$ is hydrogen, methyl or halogen, $R_1$ is hydrogen, cyano, vinyl or ethynyl, and Y is fluorine or bromine.

2. A compound according to claim 1, wherein $X_1$ is hydrogen, chlorine or methyl, $X_2$ is hydrogen or chlorine, $R_1$ is hydrogen, cyano, vinyl or ethynyl, and Y is fluorine or bromine.

3. A compound according to claim 2, wherein $X_1$ is chlorine or methyl, $X_2$ is hydrogen, $R_1$ is cyano or ethynyl, and Y is o-fluorine or p-fluorine.

4. The compound according to claim 3 of the formula

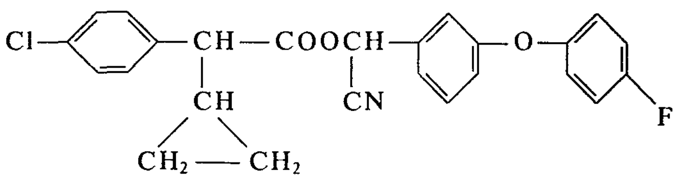

5. The compound according to claim 3 of the formula

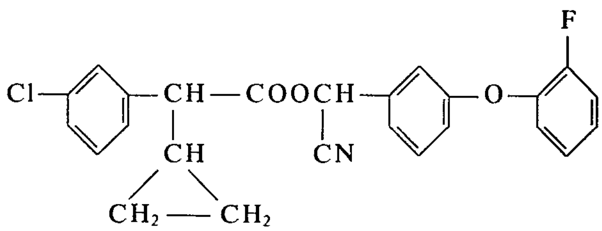

6. A process for the manufacture of a compound according to claim 1, characterised in that a compound of the formula

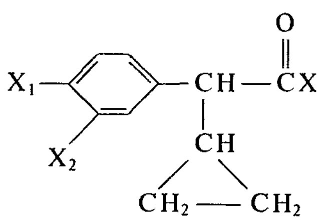

is reacted, in the presence of an acid acceptor, with a compound of the formula

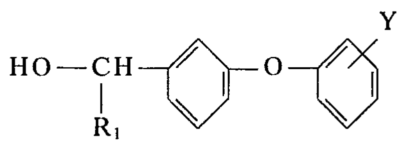

wherein $X_1$, $X_2$, Y and $R_1$ are as defined in claim 1 and X is a halogen atom.

7. A pesticidal composition which contains, as active component, a compound according to claim 1 and suitable carriers and/or other adjuvants.

8. Use of a compound according to claim 1 for controlling a variety of animal and plant pests.

9. Use according to claim 8 for controlling insects and representatives of the order Acarina.

**Claims for the contracting state: AT**

1. A pesticidal composition, characterised in that it contains an α-phenyl-α-cyclopropylacetate of the general formula

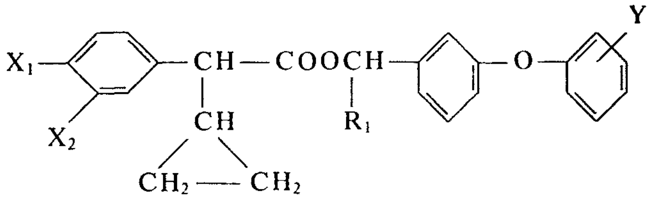

(I)

wherein $X_1$ is hydrogen, halogen or $C_1$—$C_4$-alkyl, $X_2$ is hydrogen, methyl or halogen, $R_1$ is hydrogen, cyano, vi.·yl or ethynyl, and Y is fluorine or bromine.

2. A composition according to claim 1, wherein $X_1$ is hydrogen, chlorine or methyl, $X_2$ is hydrogen or chlorine, $R_1$ is hydrogen, cyano, vinyl or ethynyl, and Y is fluorine or bromine.

3. A composition according to claim 1, wherein $X_1$ is chlorine or methyl, $X_2$ is hydrogen, $R_1$ represents cyano or ethynyl, and Y is o-fluorine or p-fluorine.

4. A composition according to claim 1 which contains, as active component, the compound of the formula

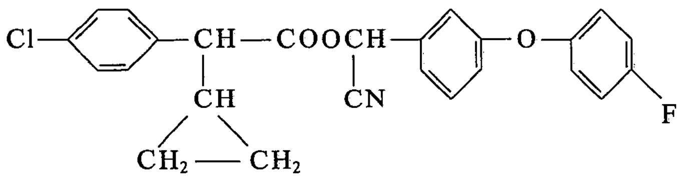

5. A composition according to claim 1 which contains, as active component, the compound of the formula

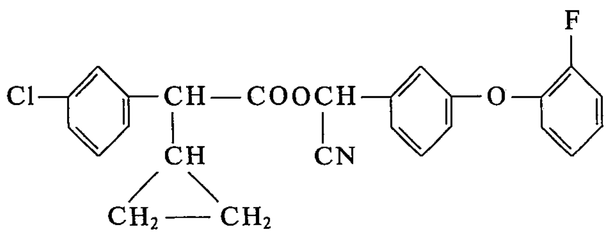

**Revendications pour les états contractants: BE, CH, DE, FR, GB, IT et NL**

1. α-phényl-α-cyclopropyl-acétate de formule:

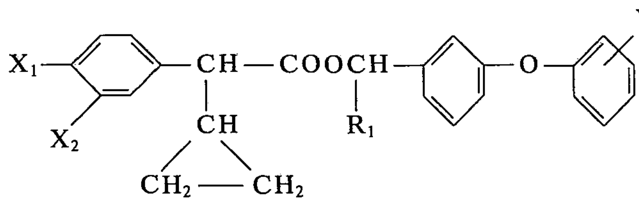

(I)

où $X_1$ représente un hydrogène, un halogène ou un alcoyle en $C_1$ à $C_4$, $X_2$ un hydrogène, un méthyle ou un halogène, $R_1$ un hydrogène, un cyano, un vinyle ou un éthynyle et Y un fluor ou un brome.

2. Composé selon la revendication 1, où $X_1$ représente un hydrogène, un chlore ou un méthyle, $X_2$ un hydrogène ou un chlore, $R_1$ un hydrogène, un cyano, un vinyle ou un éthynyle et Y un fluor ou un brome.

3. Composé selon la revendication 2, où $X_1$ représente un chlore ou un méthyle, $X_2$ un hydrogène, $R_1$ un cyano ou un éthynyle et Y un o-fluor ou un m-fluor ou un p-fluor.

4. Composé selon la revendication 3 de formule:

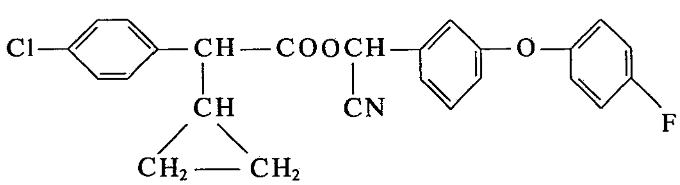

5. Composé selon la revendication 3 de formule:

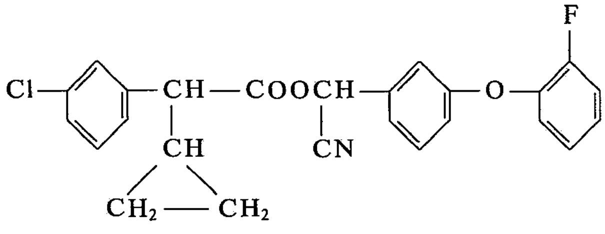

6. Procédé de préparation de composés selon la revendication 1, caractérisé en ce qu'on fait réagir un composé de formule:

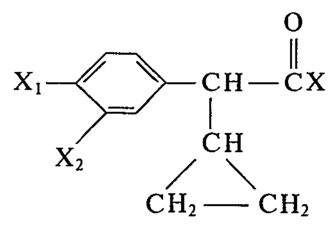

en présence d'un liant pour un acide avec un composé de formule:

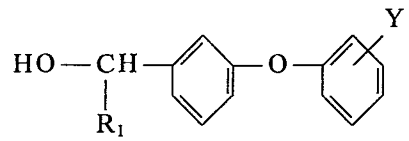

où $X_1$, $X_2$, Y et $R_1$ ont la signification donnée dans la revendication 1 et où X représente un atome d'halogène.

7. Agent de lutte antiparasitaire qui comprend comme composant actif un composé selon la revendication 1 et des supports et/ou autres additifs appropriés.

8. Application d'un composé selon la revendication 1 à la lutte contre différents types de parasites animaux et végétaux.

9. Application selon la revendication 8 à la lutte contre les insectes et les représentants de l'ordre des acariens.

**Revendications pour l'état contractant: AT**

1. Agent de lutte antiparasitaire, caractérisé en ce qu'il contient comme composant actif l'α-phényl-α-cyclopropyl-acétate de formule générale:

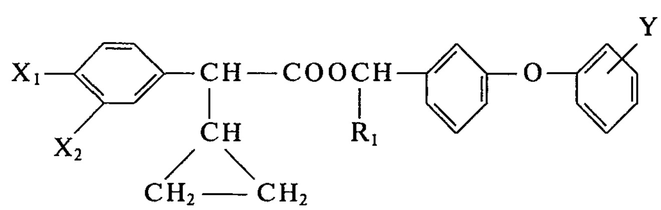

où $X_1$ représente un hydrogène, un halogène ou un alcoyle en $C_1$ à $C_4$, $X_2$ un hydrogène, un méthyle ou un halogène, $R_1$ un hydrogène, un cyano, un vinyle ou un éthynyle et Y un fluor ou un brome.

2. Agent selon la revendication 1, où $X_1$ représente un hydrogène, un chlore ou un méthyle, $X_2$ un hydrogène ou un chlore, $R_1$ un hydrogène, un cyano, un vinyle ou un éthynyle et Y un fluor ou un brome.

3. Agent selon la revendication 1, où $X_1$ représente un chlore ou un méthyle, $X_2$ un hydrogène, $R_1$ un cyano ou un éthynyle et Y un o-fluor, un m-fluor ou un p-fluor.

4. Agent selon la revendication 1 qui contient comme composant actif le composé de formule

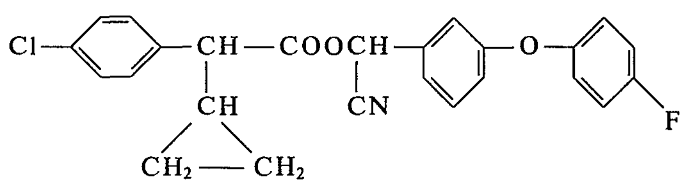

5. Agent selon la revendication 1 qui contient comme composant actif le composé de formule